## Europäisches Patentamt
## European Patent Office
## Office européen des brevets

(19)

(11) Publication number: **0 065 168**
**B1**

(12)
# EUROPEAN PATENT SPECIFICATION

(45) Date of publication of patent specification: **07.08.85**

(51) Int. Cl.⁴: $C\ 07\ D\ 201/16$, $B\ 01\ D\ 3/14$

(21) Application number: **82103753.8**

(22) Date of filing: **03.05.82**

(54) **Process for the purification of epsilon caprolactam.**

(30) Priority: **09.05.81 NL 8102280**

(43) Date of publication of application:
**24.11.82 Bulletin 82/47**

(45) Publication of the grant of the patent:
**07.08.85 Bulletin 85/32**

(84) Designated Contracting States:
**BE CH DE FR GB IT LI NL**

(56) References cited:
**EP-A-0 022 161**
**DE-A-2 035 859**
**FR-A-2 450 255**
**GB-A-1 296 670**

**CHEMICAL ENGINEERING PROGRESS, 1979,
published in New York, USA, March 1979,
pages 86-91 USA, March 1979, pages 86-91 R.F.
STRIGLE et al.: "Packed distillation column
design"**

**CHEMICAL ENGINEERING PROGRESS, 1977,
published in New York, USA, November 1977,
pages 71-77 W. MEIER et al.: "Performance of a
new, high efficiency packing"**

**CHEMIE, INGENIEUR TECHNIK, vol. 37,
published in Weinheim, DE., 1965, pages
322-328 DR. A. SPERANDIO et al.: "Eine neue
Packung für die Vakuumrektification"**

(73) Proprietor: **STAMICARBON B.V.**
**Postbus 10**
**NL-6160 MC Geleen (NL)**

(72) Inventor: **Rulkens, Peter Franciscus Maria**
**Kerkhofweg 23**
**NL-6142 BP Sittard (NL)**
Inventor: **Haasen, Nicolaas Franciscus**
**Lintjeshaag 20**
**NL-6141 MB Sittard (NL)**
Inventor: **Plantema, Otto Gerrit**
**Braakpeel 1**
**NL-6034 RP Nederweert (NL)**

(74) Representative: **Hoogstraten, Willem Cornelis
Roeland et al**
**OCTROOIBUREAU DSM Postbus 9**
**NL-6160 MA Geleen (NL)**

Courier Press, Leamington Spa, England.

## Description

The invention relates to a process for the purification of ε-caprolactam (raw material for nylon-6, e.g.) obtained through Beckmann rearrangement of cyclohexanone oxime. Such caprolactam contains several impurities which have to be removed in order to obtain a product suitable for polymerization. This is effected in a number of processing steps, usually distinguished into a pre-purification and a final purification step. The latter purification is normally performed by subjecting the caprolactam, already having a high degree of purity, e.g. 99%, to distillation under reduced pressure.

According to the German patent application No. 2035859, distillation of dehydrated liquid crude caprolactam can be carried out very effectively with the help of three film rectifiers and three film evaporators. In applying this known method of distillation for final purification of caprolactam obtained through rearrangement of cyclohexanone oxime by means of sulphuric acid or oleum, it has been found, however, that the purity of the final product obtained can be further improved, which is necessary for a number of applications of the caprolactam. According to the French patent application no. 2.450.255, purification of lactam can be carried out by means of a three stage distillation, in which in the last step an (earth)-alkalimetal hydroxide is added. The European patent application no. 22161 is concerned with the distillation of lactam distillation residue in at least two stages. A distillation process has been found now for the final purification of dehydrated caprolactam (water content less than 1% by wt.) which yields very pure caprolactam and which requires less expenses on equipment.

The process according to the invention for the purification of ε-caprolactam, obtained through rearrangement of cyclohexanone oxime with the help of sulphuric acid or oleum, by subjecting the caprolactam to be purified to distillation under reduced pressure, with separation of low-boiling and high-boiling impurities, is characterized in that the distillation is carried out in two steps with, at least in the first step, an evaporator with a rectification column containing packing material with a pressure drop of less than 2.5 mbar per theoretical tray, the caprolactam to be purified is supplied to the rectification column of the first step, the low-boiling impurities are carried off as top product from this column, the bottom product remaining in the 1st step is supplied to the 2nd step and in the latter step the high-boiling impurities are carried off as bottom product, and the purified caprolactam is recovered as top product. Preference is given to using in the second step, too, an evaporator with a rectification column containing packing material with a pressure drop of less than 2.5 mbar per theoretical tray. The said pressure drop of less than 2.5 mbar per theoretical tray relates to a measurement under standard conditions, viz. the rectification of a cis-trans decalin mixture (50% cis and 50% trans) with total reflux at a pressure of 50 mbar and a vapour rate of 5.2 m/s. (see F. J. Zuiderweg, Recommended Test Mixtures for Distillation Columns 1969, Institution of Chemical Engineers). In the distillation according to the invention several evaporators can be used, for instance a falling film evaporator. As packing material for the rectification columns any packing material is suitable which gives a pressure drop of less than 2.5 mbar per theoretical tray. Such packing material is commercially available, for instance Intalox metal packing (described in Chemical Engineering Progress, March 1979, pp. 86—91), Sulzer packing, BX type (see Chemie Ingenieur Technik, part 37, p. 322, 1965) and Sulzer packing, Mellapak type (see Chemical Engineering Progress, November 1977, pp. 71—77). Preferably a packing material is used which gives a pressure drop of less than 1.5 mbar per theoretical tray, for instance the said Mellapak type of Sulzer. The required number of theoretical trays in the rectification depends on the desired separation capacity. Usually, 5—15 theoretical trays in the 1st step and 1—5 in the 2nd step, if in this step an evaporator and a rectification column are used, are sufficient for a good result. The caprolactam to be purified is supplied to the rectification column of the 1st step either into the top of this column or into the column itself. If in the second step an evaporator and a rectification column are used, the bottom product of the 1st step can be supplied to the 2nd step either to the evaporator or to the rectification column, for instance to the 1st theoretical tray from the bottom of the column. The purity of the caprolactam to be distilled according to the invention may be different and usually will be 95—99.9 %. If the purity of the product to be distilled is lower than 95%, this product may be distilled according to the invention, if so desired, but it is recommendable to improve the pre-purification to the extent that prior to the final purification according to the invention, a starting product with a purity higher than 95% can be applied. The chosen reduced pressure at which the distillation according to the invention can be carried out is variable, in relation with the desired bottom temperatures. Preferably, a bottom pressure of 5—70 mbar is applied in the rectification column of the 1st step, and a bottom pressure of 5—15 mbar in the rectification column of the 2nd step, if in this step a rectification column with said packing material is used. The chosen bottom temperature of the rectification column in the 1st step is preferably between 115 and 175°C and in the 2nd step preferably between 115 and 145°C, if in this step a rectification column with said packing material is used. The invention will be further elucidated in the following examples:

### Example I

In a two-step vacuum distillation installation with in each step a rectification column with a condensor and a falling film evaporator for the heating of the bottom liquid, ε-caprolactam (puri-

ty 99.4% by wt.), obtained through rearrangement of cyclohexanone oxime with oleum, is purified. The falling film evaporator used is of the Normag 9318 S type. Sulzer BX type packing (pressure drop 0.7 mbar per theoretical tray) is used in the rectification columns (diameter 7 cm). A quantity of 8827 g per hour of the caprolactam to be purified (to which 0.2 mg solid NaOH per g caprolactam has been added, as usual in the purification of caprolactam by distillation) is supplied to the 5th theoretical tray (counted from the bottom) of the rectification column of the 1st step (7.5 theoretical trays). The bottom pressure of the rectification column is 12 mbar, the temperature is 141°C in the bottom, 134.5°C in the top. In the falling film evaporator of the 1st step a heating medium with a temperature of 180°C is used. In the 1st step, 466 g distillate per hour is carried off via the condensor, the reflux ratio being 3. A quantity of 1972 g per hour of the bottom product from the 1st step is supplied to the evaporator of the 2nd step. In this step the number of theoretical trays of the rectification column is 2.5, the reflux ratio 0.3, the bottom pressure in the column 8 mbar, the temperature in the top of the column 123°C and the temperature in the bottom of the column 133°C. In the falling film evaporator of the 2nd step a heating medium with a temperature of 165.5°C is used. In the 2nd step 1780 g ε-caprolactam per hour, with a purity in excess of 99.9% by wt., is carried off via the condensor.

The colour index of the purified caprolactam is 3° Hazen (50% by wt. aqueous solution, measured with a layer thickness of 4 cm with light of 290 nm, expressed as the logarithm of the portion of the light which is absorbed) is 0.11 and the permanganate number is above 10,000.

If caprolactam is purified without packing in the rectification columns (columns designed as falling film evaporators) in otherwise the same way, a product is obtained having an extinction of 0.18 and a permanganate number of 7000.

### Example II

In the distillation installation as described in Example I, 9144 g per hour of ε-caprolactam (purity 99.3% by wt.), obtained through rearrangement of cyclohexanone oxime with oleum, is supplied to the 5th theoretical tray of the rectification column of the 1st step. In the 1st step, at a bottom pressure of 10 mbar in the rectification column and a reflux ratio of 8, 331 g per hour of distillate is carried off via the condensor. The top temperature in the rectification column in the 1st step is 133°C and the bottom temperature is 142.5°C. In the falling film evaporator in the 1st step a heating medium with a temperature of 180°C is used. Of the bottom product obtained in the 1st step, a quantity of 2255 g per hour is supplied to the evaporator of the 2nd step. At a bottom pressure of 7.33 mbar in the rectification column of the second step and a reflux ratio of 0.05, 2145 g caprolactam per hour with a purity in excess of 99.9% by wt. is carried off via the condensor. The top temperature in the rectification column of the 2nd step is 122°C; the bottom temperature is 134.5°C. In the falling film evaporator of the 2nd step a heating medium with a temperature of 166°C is used. The final product obtained has a colour index of 2° Hazen, an extinction value of 0.1 and a permanganate number of more than 10,000.

### Example III

In a distillation installation of the type described in Example I, 8248 g per hour of ε-caprolactam (purity 99.5% by wt.), obtained through rearrangement of cyclohexanone oxime with oleum, is supplied to the 5th theoretical tray of the rectification column of the 1st step (10 theoretical trays). In the first step, at a bottom pressure of 58 mbar in the rectification column and a reflux ratio of 3,446 g per hour of distillate is carried off via the condensor. The top temperature in the rectification column in the 1st step is 164°C and the bottom temperature is 170°C. In the falling film evaporator in the 1st step a heating medium with a temperature of 214°C is used. Of the bottom product obtained in the 1st step, a quantity of 1848 g per hour is supplied to the evaporator of the 2nd step. At a bottom pressure of 7.38 mbar in the rectification column (1 theoretically tray) of the 2nd step and a reflux ratio of 0.3, 1752 g per hour of caprolactam with a purity in excess of 99.9% by wt. is carried off via the condensor. The top temperature in the rectification column of the 2nd step is 122°C; the bottom temperature is 130°C. In the falling film evaporator of the 2nd step a heating medium with a temperature of 170°C is used. The final product obtained has a colour index of 0 ° Hazen, an extinction value of 0.1 and a permanganate number of more than 10,000.

### Claims

1. Process for the purification of ε-caprolactam, obtained through rearrangement of cyclohexanone oxime by means of sulphuric acid or oleum, by subjecting the caprolactam to be purified to distillation under reduced pressure, with separation of low-boiling and high-boiling impurities, characterized in that the distillation is carried out in two steps with at least in the 1st step an evaporator with a rectification column containing packing material with a pressure drop of less than 2.5 mbar per theoretical tray, the caprolactam to be purified is supplied to the rectification column of the 1st step, the low-boiling impurities are carried off from this column, the bottom product remaining in the 1st step is supplied to the 2nd step and in this step the high-boiling impurities are carried off as bottom product, and the purified caprolactam is recovered as top product.

2. Process according to claim 1, characterized in that the 2nd step, too, consists of an evaporator with a rectification column containing packing material with a pressure drop of less than 2.5 mbar per theoretical tray.

3. Process according to claim 2, characterized in that a packing material with a pressure drop of less than 1.5 mbar per theoretical tray is used in the rectification columns of both steps.

4. Process according to any one of claims 1—3, characterized in that the starting material is ε-caprolactam with a purity of 95—99.9% by wt.

5. Process according to any one of claims 2—4, characterized in that a bottom pressure of 5—70 mbar is applied in the rectification column of the 1st step, and a bottom pressure of 5—15 mbar in the rectification column of the 2nd step.

6. Process according to any one of claims 2—5, characterized in that a bottom temperature of 115—175°C is applied in the rectification column of the 1st step, and a bottom temperature of 115—145°C in the rectification column of the 2nd step.

## Patentansprüche

1. Verfahren zur Reinigung von ε-Caprolactam, erhalten durch Umlagerung von Cyclohexanonoxim mittels Schwefelsäure oder Oleum, indem man das zu reinigende Caprolactam der Destillation unter vermindertem Druck unter Abtrennung der niedrigsiedenden und hochsiedenden Verunreinigungen unterwirft, dadurch gekennzeichnet, daß die Destillation in zwei Stufen mit wenigstens in der ersten Stufe einem Verdampfer mit einer Rektifizierkolonne enthaltend ein Packungsmaterial mit einem Druckabfall von weniger als 2,5 mbar pro theoretischem Boden durchgeführt wird, wobei das zu reinigende Caprolactam der Rektifizierkolonne der ersten Stufe zugeführt wird, die niedrigsiedenden Verunreinigungen aus dieser Kolonne abgezogen werden, das in der ersten Stufe verbleibende Sumpfprodukt der zweiten Stufe zugeführt wird und in dieser Stufe die hochsiedenden Verunreinigungen als Sumpfprodukt abgezogen und das Caprolactam als Kopfprodukt gewonnen wird.

2. Verfahren nach Anspruch 1, dadurch gekennzeichnet, daß die zweite Stufe ebenfalls aus einem Verdampfer mit einer Rektifizierkolonne enthaltend ein Packungsmaterial mit einem Druckabfall von weniger als 2,5 mbar pro theoretischem Boden besteht.

3. Verfahren nach Anspruch 2, dadurch gekennzeichnet, daß ein Packungsmaterial mit einem Druckabfall von weniger als 1,5 mbar pro theoretischem Boden in den Rektifizierkolonnen beider Stufen verwendet wird.

4. Verfahren nach einem der Ansprüche 1—3, dadurch gekennzeichnet, daß das Ausgangsmaterial ε-Caprolactam mit einer Reinheit von 95—99,9 Masse-% ist.

5. Verfahren nach einem der Ansprüche 1—4, dadurch gekennzeichnet, daß ein Bodendruck von 5—70 mbar in der Rektifizierkolonne der ersten Stufe und ein Bodendruck von 5—15 mbar in der Rektifizierkolonne der zweiten Stufe angewendet wird.

6. Verfahren nach einem der Ansprüche 2—5, dadurch gekennzeichnet, daß eine Bodentemperatur von 115—175°C in der Rektifizierkolonne der ersten Stufe und eine Bodentemperatur von 115—145°C in der Rektifizierkolonne der zweiten Stufe angewendet wird.

## Revendications

1. Procédé de purification de l'ε-caprolactame, obtenu par transposition de la cyclohexanone oxime au moyen d'acide sulfurique ou d'oléum, selon lequel on soumet le caprolactame à purifier à une distillation sous pression réduite, avec séparation des impuretés de points d'ébullition élevé et bas, procédé caractérisé en ce qu'on effectue la distillation en deux étapes, avec, au moins dans la première étape, un évaporateur avec une colonne de rectification contenant un matériau de garnissage avec une chute de pression inférieure à 2,5 mbar par plateau théorique, le caprolactame à purifier étant fourni à la colonne de rectification de la première étape, les impuretés de bas point d'ébullition étant éliminées de cette colonne, la fraction de queue subsistant dans la première étape étant fournie à la seconde étape et, dans cette dernière étape, les impuretés de point d'ébullition élevé étant éliminées comme fraction de queue et le caprolactame purifié étant récupéré comme fraction de tête.

2. Procédé selon la revendication 1, caractérisé en ce que la seconde étape consiste aussi en un évaporateur avec une colonne de rectification contenant un matériau de garnissage avec une chute de pression inférieure à 2,5 mbar par plateau théorique.

3. Procédé selon la revendication 2, caractérisé en ce qu'un matériau de garnissage avec une chute de pression inférieure à 1,5 mbar par plateau théorique est utilisé dans les colonnes de rectification des deux étapes.

4. Procédé selon l'une des revendications 1 à 3, caractérisé en ce que le produit de départ est un ε-caprolactame d'une pureté de 95—99,9% en poids.

5. Procédé selon l'une des revendications 2 à 4, caractérisé en ce qu'on applique une pression de 5—70 mbar dans le bas de la colonne de rectification de la première étape, et une pression de 5—15 mbar dans le bas de la colonne de rectification de la seconde étape.

6. Procédé selon l'une des revendications 2 à 5, caractérisé en ce qu'on applique une température de 115—175°C dans le bas de la colonne de rectification de la première étape, et une température de 115—145°C dans le bas de la colonne de rectification de la seconde étape.